# FASCICULE DE BREVET EUROPEEN

(11) **EP 3 389 829 B1**
(45) Date de publication et mention de la délivrance du brevet: **30.10.2019**
(21) Numéro de dépôt: 16819840.6
(22) Date de dépôt: 13.12.2016
(51) Int. Cl.: B01D 53/14, C07C 215/08, C10L 3/10

(54) **PROCÉDÉ D'ÉLIMINATION DE COMPOSÉS ACIDES D'EFFLUENT GAZEUX AVEC UNE SOLUTION ABSORBANTE A BASE DE DERIVÉS HYDROXYLES DE LA 1,6-HEXANEDIAMINE**
VERFAHREN ZUR ENTFERNUNG VON SAUERGASEN AUS EINEM GASSTROM MITTELS EINER ABSORPTIONSLÖSUNG AUF BASIS VON HYDROXYLDERIVATEN VON 1,6-HEXANDIAMIN
METHOD FOR ELIMINATING ACID COMPOUNDS FROM A GASEOUS EFFLUENT WITH AN ABSORBENT SOLUTION BASED ON HYDROXYL DERIVATIVES OF 1,6-HEXANEDIAMINE

(30) Priorité: 17.12.2015 FR 1562670
(43) Date de publication de la demande: 24.10.2018
(73) Titulaire: IFP Energies nouvelles, 92500 Rueil-Malmaison (FR)
(72) Inventeur: DELFORT, Bruno, 75005 Paris (FR); GRANDJEAN, Julien, 69008 Lyon (FR); HUARD, Thierry, 69360 Saint Symphorien D'Ozon (FR); GIRAUDON, Laetitia, 42410 Saint Michel sur Rhone (FR); LEFEBVRE, Catherine, 78230 Le Pecq (FR); WENDER, Aurélie, 92500 Rueil-Malmaison (FR); NIGON, Armelle, 92500 Rueil-Malmaison (FR)
(74) Mandataire: IFP Energies nouvelles
(86) Numéro de dépôt international: PCT/EP2016/080827
(87) Numéro de publication internationale: WO 2017/102746

(56) Documents cités:
- FR-A1- 2 934 172
- US-A1- 2003 144 371
- US-B1- 6 596 663
- US-B2- 8 877 825

## Description

### Domaine de l'invention

La présente invention concerne le domaine des procédés de désacidification d'un effluent gazeux. L'invention s'applique avantageusement au traitement de gaz d'origine industrielle et du gaz naturel.

### Contexte général

On utilise couramment des procédés de désacidification de gaz mettant en œuvre des solutions aqueuses d'amines pour retirer les composés acides présents dans un gaz, notamment le dioxyde de carbone (CO₂), l'hydrogène sulfuré (H₂S), l'oxysulfure de carbone (COS), le disulfure de carbone (CS₂), le dioxyde de soufre (SO₂) et les mercaptans (RSH) tel que le méthylmercaptan (CH₃SH), l'éthylmercaptan (CH₃CH₂SH) et le propylmercaptan (CH₃CH₂CH₂SH). Le gaz est désacidifié par mise en contact avec la solution absorbante, puis la solution absorbante est régénérée thermiquement.

Ces procédés de désacidification de gaz acides sont aussi communément appelés "lavage aux solvants" avec un solvant dit "chimique", par opposition à l'utilisation solvant dit "physique" pour l'absorption qui n'est pas basée sur la réalisation de réactions chimiques.

Un solvant chimique correspond à une solution aqueuse comprenant un réactif qui réagit sélectivement avec les composés acides (H₂S, CO₂, COS, CS₂, etc.) présents dans le gaz traité pour former des sels, sans réagir avec les autres composés non acides du gaz. Le gaz traité après mise en contact avec le solvant est alors appauvri en composés acides, lesquels sont sélectivement transférés sous forme de sels dans le solvant. Les réactions chimiques sont réversibles, ce qui permet au solvant chargé en composés acides d'être ensuite désacidifié, par exemple sous l'action de la chaleur, pour libérer d'une part les composés acides sous forme de gaz, qui peuvent alors être stockés, transformés ou être utilisés pour diverses applications, et pour d'autre part régénérer le solvant qui retourne à son état initial et peut ainsi être réutilisé pour une nouvelle phase de réaction avec le gaz acide à traiter. La phase de réaction du solvant avec le gaz acide est communément appelée la phase d'absorption, et celle où le solvant est désacidifié est appelée la phase de régénération du solvant.

En général, les performances de la séparation des composés acides du gaz, dans ce contexte, dépendent principalement de la nature de la réaction réversible choisie. Les procédés conventionnels de désacidification de gaz acides sont généralement des procédés dits "aux amines", c'est à dire qu'ils reposent sur les réactions des composés acides avec des amines en solution. Ces réactions entrent dans le cadre général des réactions acido-basiques. L'H₂S, le CO₂, ou le COS sont par exemple des composés acides, notamment en présence d'eau, alors que les amines sont des composés basiques. Les mécanismes des réactions et la nature des sels obtenus dépendent généralement de la structure des amines mises en œuvre.

Par exemple le document US 6 852 144 décrit une méthode d'élimination des composés acides des hydrocarbures utilisant une solution absorbante eau-N-méthyldiéthanolamine ou eau-triéthanolamine contenant une forte proportion d'au moins un composé appartenant au groupe suivant : pipérazine et/ou méthylpipérazine et/ou morpholine.

Les performances des procédés de désacidification de gaz acides par lavage aux amines sont directement dépendantes de la nature de la ou des amines présentes dans le solvant. Ces amines peuvent être primaires, secondaires ou tertiaires. Elles peuvent présenter une ou plusieurs fonctions amines équivalentes ou différentes par molécule.

Afin d'améliorer les performances des procédés de désacidification, il est continuellement recherché des amines toujours plus performantes.

Une limitation des solutions absorbantes couramment utilisées dans des applications de désacidification est une sélectivité insuffisante d'absorption de l'H₂S par rapport au CO₂. En effet, dans certains cas de désacidification du gaz naturel, on recherche une élimination sélective de l'H₂S en limitant au maximum l'absorption du CO₂. Cette contrainte est particulièrement importante pour des gaz à traiter contenant déjà une teneur en CO₂ inférieure ou égale à la spécification désirée. On recherche alors une capacité d'absorption de H₂S maximale avec une sélectivité maximale d'absorption de H₂S vis-à-vis du CO₂. Cette sélectivité permet de maximiser la quantité de gaz traité et de récupérer un gaz acide en sortie de régénérateur ayant une concentration la plus élevée possible en H₂S, ce qui limite la taille des unités de la chaîne soufre en aval du traitement et garantit un meilleur fonctionnement. Dans certains cas, une unité d'enrichissement en H₂S est nécessaire pour concentrer en H₂S le gaz acide. Dans ce cas, on recherche également l'amine la plus sélective. Des amines tertiaires, comme la N-méthyldiéthanolamine, ou des amines secondaires encombrées présentant une cinétique de réaction lente avec le CO₂ sont couramment utilisées, mais présentent des sélectivités limitées à des taux de charge en H₂S élevés.

Il est bien connu de l'homme du métier que les amines tertiaires ou les amines secondaires avec un encombrement stérique sévère ont une cinétique de captage du CO₂ plus lente que des amines primaires ou secondaires peu encombrées. En revanche, les amines tertiaires ou secondaires avec un encombrement stérique sévère ont une cinétique de captage de l'H₂S instantanée, ce qui permet de réaliser une élimination sélective de l'H₂S basée sur des performances cinétiques distinctes.

Différents documents proposent d'utiliser des amines tertiaires ou secondaires encombrées, en particulier des diamines tertiaires ou secondaires encombrées, en solution pour désacidifier des gaz acides.

Parmi les applications des amines tertiaires ou secondaires avec un encombrement stérique sévère, le brevet US 4,405,582 décrit un procédé d'absorption sélective de gaz sulfurés par un absorbant contenant un diaminoéther dont au moins une fonction amine est tertiaire et dont l'autre fonction amine est tertiaire ou secondaire possédant un encombrement stérique sévère, l'atome d'azote étant dans ce dernier cas lié soit à au moins un carbone tertiaire, soit à deux atomes de carbone secondaires. Les deux fonctions amines et les carbones de la chaîne principale peuvent être substitués par des radicaux alkyles ou hydroxyalkyles.

Le brevet US 4,405,583 décrit également un procédé d'élimination sélective de l'H₂S dans des gaz contenant de l'H₂S et du CO₂ par un absorbant contenant un diaminoéther dont les deux fonctions amines secondaires présentent un encombrement stérique sévère défini comme précédemment. Les substituants des fonctions amines et des carbones de la chaîne principale peuvent être substitués par des radicaux alkyle et hydroxyalkyle.

La demande de brevet FR 2 934 172 A1 divulgue un ultérieur procédé de l'art antérieur. Une solution absorbante comprenant de l'eau et de la N,N,N',N'-tétraméthylhexane-1,6-diamine y est employée. Une autre limitation des solutions absorbantes couramment utilisées dans des applications de désacidification totale est une cinétique de captage du CO₂ ou du COS trop lente. Dans le cas où les spécifications désirées sur le CO₂ ou en COS sont très poussées, on recherche une cinétique de réaction la plus rapide possible de manière à réduire la hauteur de la colonne d'absorption. Cet équipement sous pression représente en effet une part importante des coûts d'investissements du procédé.

Que l'on recherche une cinétique de captage du CO₂ et du COS maximale dans une application désacidification totale, ou une cinétique de captage du CO₂ minimale dans une application sélective, on désire toujours utiliser une solution absorbante ayant la capacité cyclique la plus grande possible. Cette capacité cyclique, notée Δα correspond à la différence de taux de charge (α désignant le nombre de mole de composés acides absorbés n_{gaz acide} par kilogramme de solution absorbante) entre la solution absorbante soutirée en fond de la colonne d'absorption et la solution absorbante alimentant ladite colonne. En effet, plus la solution absorbante a une forte capacité cyclique, plus le débit de solution absorbante qu'il faut mettre en œuvre pour désacidifier de gaz à traiter est restreint. Dans les procédés de traitement de gaz, la réduction du débit de solution absorbante a également un fort impact sur la réduction des investissements, notamment au niveau du dimensionnement de la colonne d'absorption.

Un autre aspect primordial des opérations de traitement de gaz ou fumées industrielles par solvant reste la régénération de l'agent de séparation. En fonction du type d'absorption (physique et/ou chimique), on envisage généralement une régénération par détente, et/ou par distillation et/ou par entraînement par un gaz vaporisé appelé "gaz de strippage". La consommation énergétique nécessaire à la régénération du solvant peut être très importante, ce qui est particulièrement vrai dans le cas où la pression partielle de gaz acides est faible, et représenter un coût opératoire considérable pour le procédé de captage du CO₂.

Il est bien connu de l'homme du métier que l'énergie nécessaire à la régénération par distillation d'une solution d'amine peut se décomposer selon trois postes différents : l'énergie nécessaire pour réchauffer la solution absorbante entre la tête et le fond du régénérateur, l'énergie nécessaire pour abaisser la pression partielle de gaz acide dans le régénérateur par vaporisation d'un gaz de strippage, et enfin l'énergie nécessaire pour casser la liaison chimique entre l'amine et le CO₂.

Ces deux premiers postes sont proportionnels aux débits de solution absorbante qu'il est nécessaire de faire circuler dans l'unité pour réaliser une spécification donnée. Pour diminuer la consommation énergétique associée à la régénération du solvant, il est donc préférable encore une fois de maximiser la capacité cyclique du solvant. En effet, plus la solution absorbante a une forte capacité cyclique, plus le débit de solution absorbante qu'il faut mettre en œuvre pour désacidifier de gaz à traiter est restreint.

Il existe ainsi un besoin, dans le domaine de la désacidification de gaz, de fournir des composés qui soient de bons candidats pour l'élimination des composés acides d'un effluent gazeux, notamment, mais pas exclusivement, pour l'élimination sélective de l'H₂S par rapport au CO₂, et qui permettent de fonctionner à moindres coûts opératoires (dont l'énergie de régénération) et d'investissements (dont le coût de la colonne d'absorption).

### Description de l'invention

Les inventeurs ont mis en évidence que les diamines tertiaires ou secondaires possédant un encombrement stérique sévère ne sont pas équivalentes en terme de performances pour leur usage dans des formulations de solutions absorbantes pour le traitement de gaz acides dans un procédé industriel.

La présente invention a pour objet l'utilisation, dans le domaine de la désacidification de gaz, de diamines de formule générale (I) donnée plus bas en solution aqueuse, qui sont des diamines, qui sont des dérivés hydroxyles de la 1-6,hexanediamine.

Les inventeurs ont mis en évidence que l'utilisation de ces composés permet d'obtenir de bonnes performances en terme de capacité cyclique d'absorption des gaz acides et de sélectivité d'absorption vis-à-vis de l'H₂S, notamment une sélectivité d'absorption vis-à-vis de l'H₂S plus importante que des amines de référence telles que la N-méthyldiéthanolamine (MDEA) pour une capacité cyclique d'absorption des gaz acides équivalente ou supérieure.

### Résumé de l'invention

L'invention porte, selon un premier aspect, sur une solution absorbante pour éliminer des composés acides contenus dans un effluent gazeux au moyen d'un procédé selon l'invention, cette solution absorbante comportant :
- de l'eau ;
- au moins un composé azoté répondant à la formule générale (I) suivante : 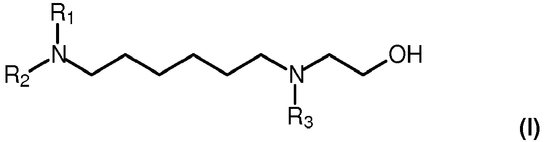 dans laquelle :
   les radicaux R₁, R₂, R₃ sont chacun choisi indifféremment parmi un radical méthyle et un radical hydroxyéthyle, et
   au moins un radical parmi R₁, R₂, et R₃ est un radical méthyle.
   Selon l'invention, le composé azoté peut être choisi parmi les composés suivants :
- la N,N,N'-triméthyl-N'-(2-hydroxyéthyl)-1,6-hexanediamine de formule (I₁) suivante : 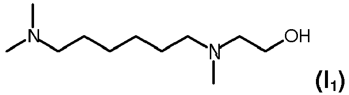
- la N,N'-diméthyl-N,N'-di(2-hydroxyéthyl)-1,6-hexanediamine de formule (I₂) : 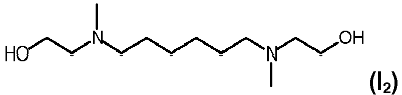
- la N,N-diméthyl-N',N'-di(2-hydroxyéthyl)-1,6-hexanediamine de formule (I₃) : 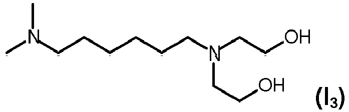
- la N-méthyl-N,N',N'-tri(2-hydroxyéthyl)-1,6-hexanediamine de formule (I₄) :

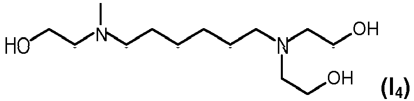

De préférence, le composé azoté est la N,N,N'-triméthyl-N'-(2-hydroxyéthyl)-1,6-hexanediamine de formule **(I₁)** ou la N,N'-diméthyl-N,N'-di(2-hydroxyéthyl)-1,6-hexanediamine de formule **(I₂).**

Selon l'invention, la solution absorbante peut comporter entre 5 % et 95 % poids du composé azoté, de préférence entre 10 % et 90 % poids du composé azoté, et entre 5 % et 95 % poids d'eau, et de préférence entre 10 % et 90% poids d'eau.

La solution comporte avantageusement entre 20 % et 50 % poids dudit composé azoté, et entre 50 % et 80 % poids d'eau.

La solution absorbante peut comporter en outre entre 5 % et 95 % poids d'au moins une amine supplémentaire, l'amine supplémentaire étant soit une amine tertiaire, soit une amine secondaire comprenant deux carbones secondaires en alpha de l'atome d'azote ou au moins un carbone tertiaire en alpha de l'atome d'azote.

L'amine supplémentaire peut être une amine tertiaire choisie dans le groupe constitué par :
- la N-méthyldiéthanolamine ;
- la triéthanolamine ;
- la diéthylmonoéthanolamine ;
- la diméthylmonoéthanolamine ; et
- l'éthyldiéthanolamine.

La solution absorbante peut comporter, en outre, une quantité non nulle et inférieure à 30 % poids d'au moins une amine primaire ou secondaire.

L'amine primaire ou secondaire peut être choisie dans le groupe constitué par :
- la monoéthanolamine ;
- la diéthanolamine ;
- la N-butyléthanolamine ;
- l'aminoéthyléthanolamine ;
- le diglycolamine ;
- la pipérazine ;
- la 1-méthyl-pipérazine ;
- la 2-méthyl-pipérazine ;
- l'homopipérazine ;
- la N-(2-hydroxyéthyl)pipérazine ;
- la N-(2-aminoéthyl)pipérazine ;
- la morpholine ;
- la 3-(méthylamino)propylamine ;
- la 1,6-hexanediamine ;
- la N,N-diméthyl-1,6-hexanediamine ;
- la N,N'-diméthyl-1,6-hexanediamine
- la N-méthyl-1,6-hexanediamine ; et
- la N,N',N'-triméthyl-1,6-hexanediamine.

La solution absorbante peut comporter en outre au moins un solvant physique choisi dans le groupe constitué par le méthanol, l'éthanol, le 2-éthoxyéthanol, le triéthylèneglycoldiméthyléther, le tétraéthylèneglycoldiméthyléther, le pentaéthylèneglycoldiméthyléther, l'hexaéthylèneglycoldiméthyléther, l'heptaéthylèneglycoldiméthyléther, l'octaéthylèneglycoldiméthyléther, le butoxyacétate de diéthylèneglycol, le triacétate de glycérol, le sulfolane, la N-méthylpyrrolidone, la N-méthylmorpholin-3-one, le N,N-diméthylformamide, la N-formyl-morpholine, la N,N-diméthyl-imidazolidin-2-one, le N-méthylimidazole, l'éthylèneglycol, le diéthylèneglycol, le triéthylèneglycol, le thiodiglycol, le carbonate de propylène, le tributylphosphate.

L'invention porte sur un procédé d'élimination des composés acides contenus dans un effluent gazeux dans lequel on effectue une étape d'absorption des composés acides par mise en contact de l'effluent gazeux avec une solution absorbante selon l'invention.

De préférence, l'étape d'absorption des composés acides est réalisée à une pression comprise entre 1 bar et 200 bar, et à une température comprise entre 20°C et 100°C.

De préférence, on obtient une solution absorbante chargée en composés acides après l'étape d'absorption, et on effectue au moins une étape de régénération de la solution absorbante chargée en composés acides à une pression comprise entre 1 bar et 10 bar et à une température comprise entre 100 °C et 180 °C.

L'effluent gazeux peut être choisi parmi le gaz naturel, les gaz de synthèse, les fumées de combustion, les gaz de raffinerie, les gaz acides issus d'une unité aux amines, les gaz issus d'une unité de réduction en queue du procédé Claus, les gaz de fermentation de biomasse, les gaz de cimenterie, les fumées d'incinérateur.

Le procédé selon l'invention peut être mis en œuvre pour éliminer sélectivement de l'H₂S par rapport au CO₂ d'un effluent gazeux comportant de l'H₂S et du CO₂.

D'autres objets et avantages de l'invention apparaîtront à la lecture de la description qui suit d'exemples de réalisations particuliers de l'invention, donnés à titre d'exemples non limitatifs, la description étant faite en référence à la figure annexée décrite ci-après.

### Brève description de la figure

La figure 1 représente un schéma de principe de mise en œuvre d'un procédé de traitement de gaz acides.
Les figures 2A et 2B illustrent un procédé de synthèse de composés de formule générale (I) à partir de la 1,6-hexanediamine selon deux variantes.
Les figures 3 à 7 représentent des voies de synthèse de la N,N'-diméthyl-N-N'-di(2-hydroxyéthyl)-1,6-hexanediamine (formule (I₂)) à partir respectivement de 1,6-hexanediol, d'adipaldéhyde, d'adipate de diméthyle ou d'acide adipique en deux étapes, d'adipate de diméthyle ou d'acide adipique en trois étapes.
La figure 8 représente une voie de synthèse de composés de formule générale (I) à partir d'un 1,6-dihalohexane.

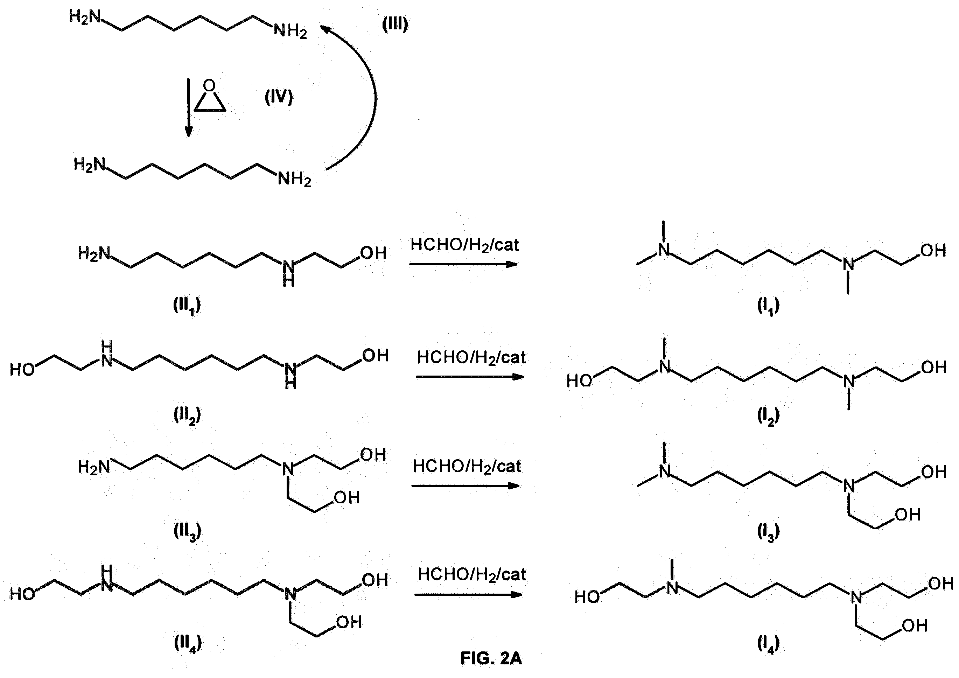

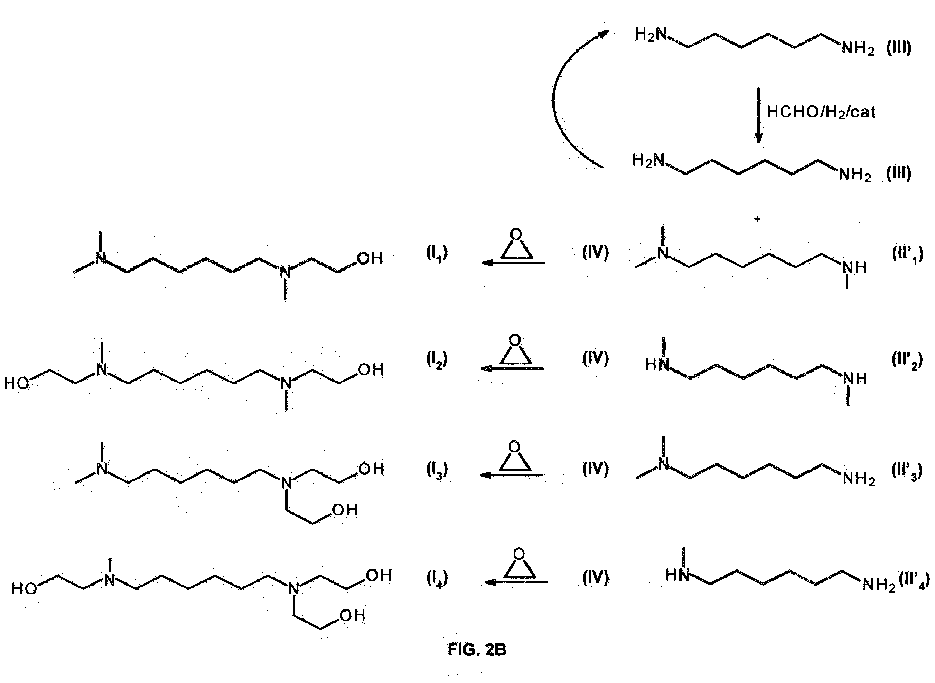

Dans les figures 2 à 8, l'abréviation "cat" signifie catalyseur, et les flèches représentent des étapes de réaction. Il s'agit de schémas réactionnels, ne comportent pas l'ensemble des composantes nécessaires à la mise en œuvre de la synthèse. Seuls les éléments nécessaires à la compréhension de l'invention y sont représentés.

### Description détaillée de l'invention

La présente invention propose d'éliminer les composés acides d'un effluent gazeux en mettant en œuvre en solution aqueuse dont la composition est détaillée ci-après.

### Composition de la solution absorbante

La solution absorbante mise en œuvre pour l'élimination des composés acides contenus dans un effluent gazeux comporte:
- de l'eau;
- au moins un composé azoté répondant à la formule générale (I) suivante : 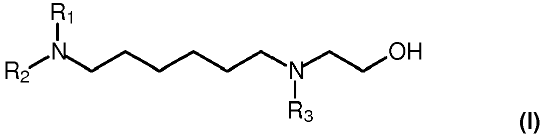 dans laquelle :
   Les radicaux R₁, R₂, R₃ sont chacun choisi indifféremment parmi un radical méthyle et un radical hydroxyéthyle, et au moins un radical parmi R₁, R₂, et R₃ est un radical méthyle.

De manière préférée, les radicaux R₁ et R₃ sont des radicaux méthyle et le radical R₂ est un radical méthyle ou hydroxyéthyle.

De manière encore plus préférée, les radicaux R₁ et R₃ sont des radicaux méthyle et le radical R₂ est un hydroxyéthyle.

La solution absorbante selon l'invention peut comporter avantageusement au moins un des composés suivants :
- la N,N,N'-triméthyl-N'-(2-hydroxyéthyl)-1,6-hexanediamine de formule **(I₁)** suivante : 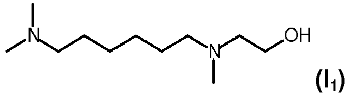
- la N,N'-diméthyl-N,N'-di(2-hydroxyéthyl)-1,6-hexanediamine de formule **(I₂)** suivante : 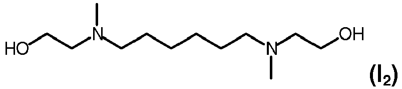
- la N,N-diméthyl-N',N'-di(2-hydroxyéthyl)-1,6-hexanediamine de formule **(I₃)** suivante : 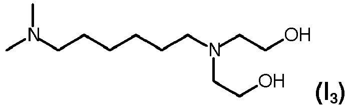
- la N-méthyl-N,N',N'-tri(2-hydroxyéthyl)-1,6-hexanediamine de formule **(I₄)** suivante :

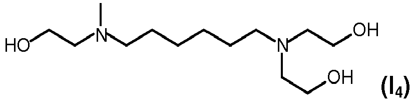

Le composé azoté de formule générale (I) peut être en concentration variable dans la solution absorbante, par exemple comprise entre 5 % et 95 % poids, de préférence entre 10 % et 90 % poids, encore plus préférentiellement entre 20 % et 50 % poids, et bornes incluses.

La solution absorbante peut contenir entre 5 % et 95 % poids d'eau, de préférence entre 10 % et 90 % poids d'eau, plus préférentiellement entre 50 % et 80 % poids d'eau, bornes incluses.

La somme des fractions massiques exprimées en % poids des différents composés de la solution absorbante est égale à 100 % en poids de la solution absorbante.

Selon un mode de réalisation, la solution absorbante peut contenir en outre au moins une amine supplémentaire qui est une amine tertiaire telle que la N-méthyldiéthanolamine, la triéthanolamine, la diéthylmonoéthanolamine, la diméthylmonoéthanolamine, ou l'éthyldiéthanolamine, ou qui est une amine secondaire ayant un encombrement stérique sévère, cet encombrement étant défini soit par la présence de deux carbones secondaires en alpha de l'atome d'azote, soit par au moins un carbone tertiaire en alpha de l'atome d'azote. On entend par ladite amine supplémentaire tout composé possédant au moins une fonction amine tertiaire ou secondaire sévèrement encombrée. La concentration de ladite amine supplémentaire tertiaire ou secondaire sévèrement encombrée dans la solution absorbante peut être comprise entre 5 % et 95 % poids, de préférence entre 5 % et 50 % poids, de manière très préférée entre 5 % et 30 % poids.

Selon un mode de réalisation, les amines selon la formule générale (I) peuvent être formulées avec un ou plusieurs composés contenant au moins une fonction amine primaire ou secondaire. Par exemple, la solution absorbante comporte jusqu'à une concentration de 30 % poids, de préférence inférieure à 15 % poids, de préférence inférieure à 10 % poids dudit composé contenant au moins une fonction amine primaire ou secondaire. De préférence, la solution absorbante comporte au moins 0,5 % poids dudit composé contenant au moins une fonction amine primaire ou secondaire. Ledit composé permet d'accélérer la cinétique d'absorption du CO₂ et, dans certains cas, du COS contenu dans le gaz à traiter.

Une liste non exhaustive de composés contenant au moins une fonction amine primaire ou secondaire qui peuvent entrer dans la formulation est donnée ci-dessous :
- la monoéthanolamine ;
- la diéthanolamine ;
- la N-butyléthanolamine ;
- l'aminoéthyléthanolamine ;
- le diglycolamine ;
- la pipérazine ;
- la 1-méthylpipérazine ;
- la 2-méthylpipérazine ;
- l'homopipérazine ;
- la N-(2-hydroxyéthyl)pipérazine ;
- la N-(2-aminoéthyl)pipérazine ;
- la morpholine ;
- la 3-(méthylamino)propylamine ;
- la 1,6-hexanediamine et tous ses dérivés diversement N-alkylés tels par exemple la N,N'-diméthyl-1,6-hexanediamine, la N,N'-diméthyl-1,6-hexanediamine, la N-méthyl-1,6-hexanediamine ou la N,N',N'-triméthyl-1,6-hexanediamine.

Selon l'invention, la solution absorbante comprenant un composé azoté de formule générale (I) peut comprendre un mélange d'amines supplémentaires tels que définies ci-dessus.

Selon un mode de réalisation, la solution absorbante peut contenir des composés organiques non réactifs vis à vis des composés acides (couramment nommé "solvants physiques"), qui permettent d'augmenter la solubilité d'au moins un ou plusieurs composés acides de l'effluent gazeux. Par exemple, la solution absorbante peut comporter entre 5% et 50% poids de solvant physique tel que des alcools, des éthers, des étheralcools, des éthers de glycol et de polyéthylèneglycol, des thioéthers de glycol, des esters et alkoxyesters de glycol et de polyéthylèneglycol, des esters de glycérol, des lactones, des lactames, des pyrrolidones N-alkylées, des dérivés de la morpholine, de la morpholin-3-one, des imidazoles et des imidazolidinones, des pipéridones N-alkylées, des cyclotétraméthylènesulfones, des N-alkylformamides, des N-alkylacétamides, des éthers-cétones des carbonates d'alkyles ou des phosphates d'alkyles et leur dérivés. A titre d'exemple et de façon non limitative, il peut s'agir du méthanol, de l'éthanol, du 2-éthoxyéthanol, du triéthylèneglycoldiméthyléther, du tétraéthylèneglycoldiméthyléther, du pentaéthylèneglycoldiméthyléther, de l'hexaéthylèneglycoldiméthyléther, de l' heptaéthylèneglycoldiméthyléther, de l'octaéthylèneglycoldiméthyléther, du butoxyacétate de diéthylèneglycol, du triacétate de glycérol, du sulfolane, de la N-méthylpyrrolidone, de la N-méthylmorpholin-3-one, du N,N-diméthylformamide, de la N-formyl-morpholine, de la N,N-diméthyl-imidazolidin-2-one, du N-méthylimidazole, de l'éthylèneglycol, du diéthylèneglycol, du triéthylèneglycol, du thiodiglycol, du carbonate de propylène, du tributylphosphate..

### Synthèse des composés azotés de la formule générale

Les composés répondant à la formule générale de l'invention peuvent être synthétisés selon toutes les méthodes permises par la chimie organique et notamment par les méthodes suivantes.

### Méthode 1 : Synthèse à partir de la 1,6-hexanediamine

Une première méthode consiste en la succession de deux étapes de réactions à partir de 1,6-hexanediamine.

La figure 2A illustre la synthèse d'au moins un composé de formule générale (I) à partir de la 1,6-hexanediamine, de formule **(III).**

La première étape consiste à faire réagir la 1,6-hexanediamine avec de l'oxyde d'éthylène (formule **(IV)** en quantité et dans des conditions adéquates afin d'obtenir soit une 1,6-hexanediamine partiellement éthoxylée telle la N-(2-hydroxyéthyl)-1,6-hexanediamine (de formule **II₁**) ou la N-N'-di(2-hydroxyéthyl)-1,6-hexanediamine (de formule **II₂**) ou la N,N-di(2-hydroxyéthyl)-1,6-hexanediamine (de formule **II₃**) ou la N-N-N'-tri(2-hydroxyéthyl)-1,6-hexanediamine (de formule **II₄**), soit un mélange de ces molécules. Ces 1,6-hexanediamines partiellement éthoxylées, de formule (**II₁**) à (**II₄**) sont des produits intermédiaires dans ce mode de synthèse.

Les conditions de réaction sont adaptées afin de ne pas obtenir de N,N,N',N'-tétra(2-hydroxyéthy)-1,6-hexanediamine et de minimiser la quantité de 1,6-hexanediamine résiduelle. Pour cela, il est avantageux d'opérer avec un rapport molaire d'oxyde d'éthylène par rapport à la 1,6-hexanediamine n'excédant pas 3/1, et de préférence n'excédant pas 2,5/1. Le choix du rapport molaire entre l'oxyde d'éthylène et la 1,6-hexanediamine conditionne la composition des produits obtenus.

Lorsque de la 1,6-hexanediamine résiduelle est présente, elle peut être éliminée du milieu par exemple par distillation et éventuellement recyclée.

Lorsque de la N,N,N',N'-tétra(2-hydroxyéthy)-1,6-hexanediamine est obtenue, elle peut être éliminée du milieu par exemple par distillation.

Lorsqu'un mélange de composés intermédiaires est obtenu, il peut être utilisé tel quel pour effectuer la seconde étape ou bien il peut être soumis à une séparation, par exemple par distillation, afin d'obtenir un des composés selon la formule générale (I) ou une combinaison de ces composés (formules **I₁** à **I₄**) à l'issue de la deuxième étape.

La deuxième étape consiste en la méthylation des fonctions amines primaires ou secondaires des composés intermédiaires (de formules **II₁** à **II₄**) obtenus lors de la première étape pour conduire à la N,N,N'-triméthyl-N'-(2-hydroxyéthyl)-1,6-hexanediamine (formule **I₁**) et/ou à la N,N'-diméthyl-N-N'-di(2-hydroxyéthyl)-1,6-hexanediamine (formule **I₂**) et/ou à la N,N-diméthyl-N'-N'-di(2-hydroxyéthyl)-1,6-hexanediamine (formule **I₃**) et/ou à la N-méthyl-N-N'-N'-tri(2-hydroxyéthyl)-1,6-hexanediamine (formule **I₄**).

La méthylation des fonctions amines peut s'effectuer par tous les moyens connus de l'homme du métier notamment par réaction de formaldéhyde et d'hydrogène en présence d'un catalyseur approprié (tel que représenté sur la figure 2A) ou par exemple par réaction de formaldéhyde et d'acide formique selon la réaction dite de Eichweiller-Clarke.

Selon une voie alternative de synthèse à partir de la de 1,6-hexanediamine, les étapes d'éthoxylation et de méthylation peuvent être inversées par rapport à la voie décrite précédemment et illustrée à la figure 2A. Dans ce cas, une première étape de méthylation partielle de la 1,6-hexanediamine, par réaction avec du formaldéhyde et de l'hydrogène en présence d'un catalyseur d'hydrogénation, conduit à la N-méthyl-1,6-hexanediamine (formule (**II**'₄)) et/ou à la N,N-diméthyl-1,6-hexanediamine (formule (**II**'₃)) et/ou à la N,N'-diméthyl-1,6-hexanediamine (formule (**II**'₂)) et/ou à la N,N,N'-triméthyl-1,6-hexanediamine (formule (**II**'₁)). Puis dans une deuxième étape les fonctions amines primaires et secondaires sont éthoxylées par réaction avec de l'oxyde d'éthylène (formule (**IV**)). Cette variante de cette première méthode de synthèse est illustrée à la figure 2B.

### Méthode 2 : Synthèse à partir de la 1,6-hexanediol

Un composé de formule générale **(I)** peut être synthétisé à partir du 1,6-hexanediol (formule **(V)**).

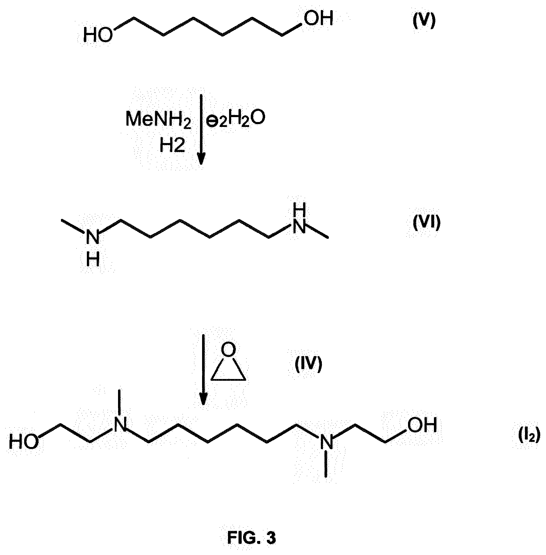

La figure 3 illustre la synthèse d'un exemple de composé selon la formule générale (I), la N,N'-diméthyl-N,N'-di(2-hydroxyéthyl)-1,6-hexanediamine de formule (**I**₂), à partir de la 1,6-hexanediol de formule **(V).**

La synthèse consiste en la succession de deux étapes de réactions : la condensation du 1,6-hexanediol avec de la méthylamine (MeNH₂) utilisée généralement en excès en présence d'hydrogène et d'un catalyseur approprié pour conduire au N,N'-diméthyl-1,6-hexanediamine (formule **(VI)**) qui est ensuite transformé en N,N'-diméthyl-N-N'-di(2-hydroxyéthyl)-1,6-hexanediamine (formule (**I**₂)) par une réaction d'éthoxylation avec de l'oxyde d'éthylène (formule **(IV)**).

### Méthode 3 : Synthèse à partir de l'adipaldéhyde

Un composé de formule générale (I) peut être synthétisé à partir d'adipaldéhyde.

### Synthèse en 3 étapes de réactions

Un composé de formule générale **(I)** peut être synthétisé en trois étapes de réactions à partir de l'adipaldéhyde (formule **(VII)**).

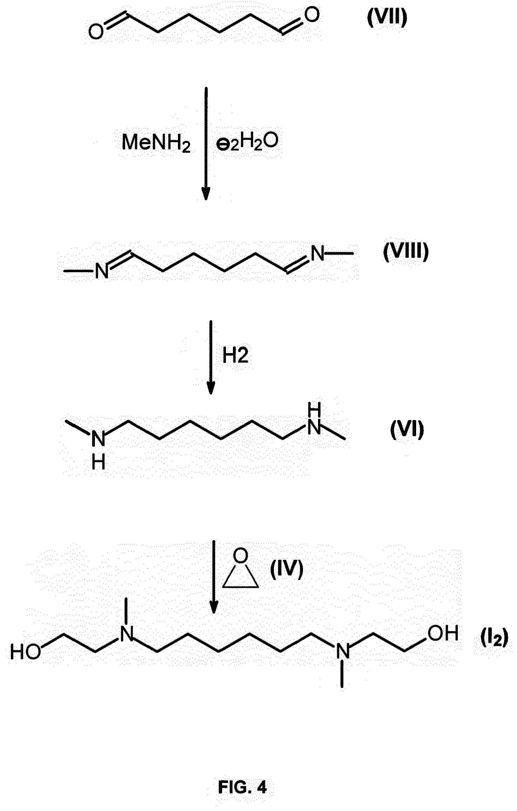

La figure 4 illustre la synthèse d'un exemple de composé selon la formule générale (I), la N,N'-diméthyl-N,N'-di(2-hydroxyéthyl)-1,6-hexanediamine de formule (**I**₂), à partir de l'adipaldéhyde de formule **(VII)**, selon un procédé de synthèse en trois étapes réactionnelles.

La synthèse consiste en la succession de trois étapes de réactions.

La première étape consiste en une réaction condensation de l'adipaldéhyde (formule **(VII)**) avec de la méthylamine pour conduire au 1,6-bis(methylimino)-hexane (formule **(VIII)**).

Une deuxième étape de réaction consiste en la réduction du 1,6-bis(methylimino)-hexane (formule **(VIII)**) en N,N'-diméthyl-1,6-hexanediamine (formule **(VI)**).

Une troisième étape de réaction consiste en l'éthoxylation de la N,N'-diméthyl-1,6-hexanediamine (formule **(VI)**) avec de l'oxyde d'éthylène (formule **(IV)**) pour former de la N,N'-diméthyl-N-N'-di(2-hydroxyéthyl)-1,6-hexanediamine (formule **(I₂)**).

### Synthèse en 1 étape de réaction

Un composé de formule générale (I) peut être synthétisé en une seule étape de à partir d'adipaldéhyde.

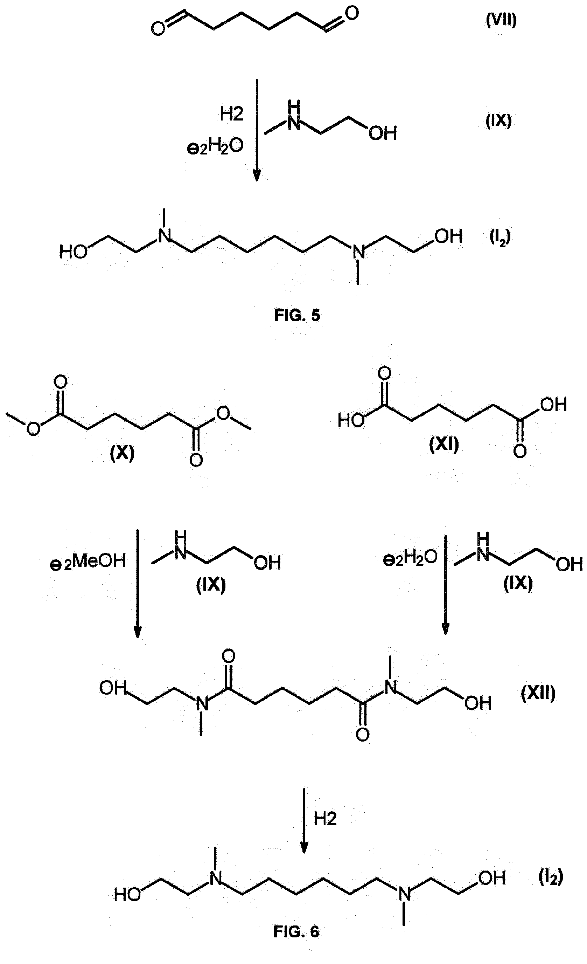

La figure 5 illustre la synthèse d'un exemple de composé selon la formule générale (I), la N,N'-diméthyl-N,N'-di(2-hydroxyéthyl)-1,6-hexanediamine de formule **(I₂)**, à partir de l'adipaldéhyde (formule **(VII)**), selon un procédé de synthèse en une étape réactionnelle.

La synthèse consiste en la condensation de l'adipaldéhyde (formule **(VII)**) avec le N-méthyl-2-aminoéthanol (formule **(IX)**) en présence d'hydrogène et d'un catalyseur d'hydrogénation adéquat pour conduire à la N,N'-diméthyl-N-N'-di(2-hydroxyéthyl)-1,6-hexanediamine (formule **(I₂)**).

Cette transformation, aussi appelée amination réductrice, résulte de la succession de deux réactions qui sont la condensation d'une amine secondaire avec un aldéhyde conduisant à une enamine, puis l'hydrogénation en amine tertiaire de cette dernière.

### Méthode 4 : Synthèse à partir de l'acide adipique ou d'un diester de l'acide adipique

Un composé de formule générale (I) peut être synthétisé à partir de l'acide adipique ou d'un diester de l'acide adipique.

### Synthèse en 2 étapes de réactions

Un composé de formule générale **(I)** peut être synthétisé en deux étapes de réactions à partir de l'acide adipique (formule **(XI)**) ou d'un diester de l'acide adipique tel que l'adipate de diméthyle (formule **(X)**).

La figure 6 illustre la synthèse d'un exemple de composé selon la formule générale (I), la N,N'-diméthyl-N,N'-di(2-hydroxyéthyl)-1,6-hexanediamine de formule **(I₂)**, à partir de de l'acide adipique (formule **(XI)**) ou d'un diester de l'acide adipique, tel que l'adipate de diméthyle (formule **(X)**), selon un procédé de synthèse en deux étapes réactionnelles.

Une première étape consiste en la réaction de condensation de N-méthyl-2-aminoéthanol (formule **(IX)**) avec soit de l'acide adipique (formule **(XI)**) soit un diester de l'acide adipique tel par exemple sans être limitatif l'adipate de diméthyle (formule **(X)**) ou l'adipate de diéthyle. Le produit de condensation obtenu est un diamide le N,N'-diméthyl-N,N'-di(2-hydroxyéthyl)-adipamide (formule **(XII)**).

La deuxième étape consiste en la réduction des fonctions amides du N,N'-diméthyl-N,N'-di(2-hydroxyéthyl)-adipamide (formule **(XII)**) en fonctions amines tertiaires pour conduire à la N,N'-diméthyl-N-N'-di(2-hydroxyéthyl)-1,6-hexanediamine (formule **(I₂)**).

Cette réduction peut s'effectuer par tous les moyens connus telle la réduction par hydrogénation catalytique ou l'action d'hydrures tels l'hydrure de lithium et d'aluminium.

### Synthèse en 3 étapes de réactions

Un composé de formule générale **(I)** peut être synthétisé en trois étapes de réactions à partir de l'acide adipique (formule **(XI)**) ou d'un diester de l'acide adipique tel que l'adipate de diméthyle (formule **(X)**).

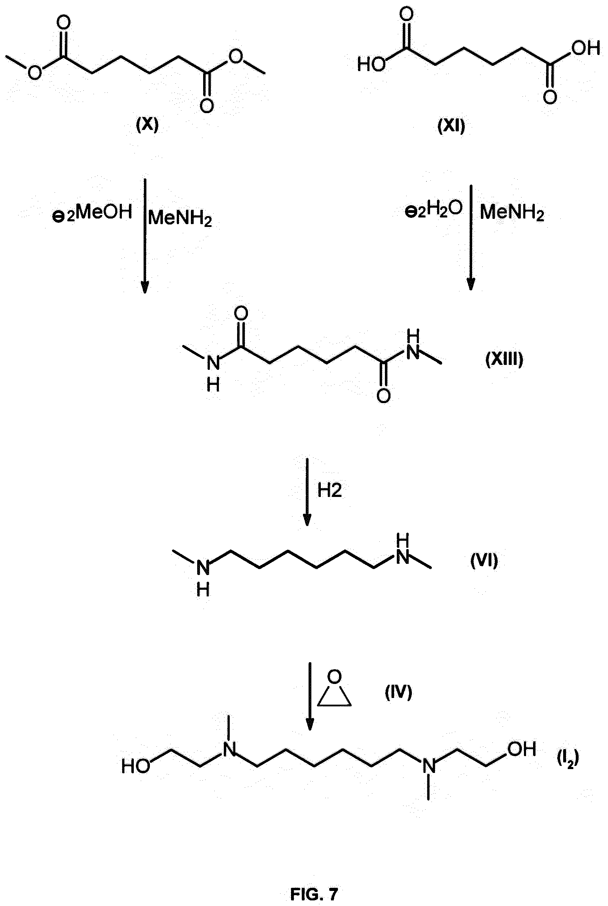

La figure 7 illustre la synthèse d'un exemple de composé selon la formule générale (I), la N,N'-diméthyl-N,N'-di(2-hydroxyéthyl)-1,6-hexanediamine de formule **(I₂)**, à partir de de l'acide adipique (formule **(XI)**) ou d'un diester de l'acide adipique, tel que l'adipate de diméthyle (formule **(X)**)**,** selon un procédé de synthèse en trois étapes réactionnelles.

Une première étape consiste en la condensation de la méthylamine (MeNH2) avec soit de l'acide adipique (formule **(XI)**) soit un diester de l'acide adipique tel par exemple sans être limitatif l'adipate de diméthyle (formule **(X)**) ou l'adipate de diéthyle. Le produit de condensation obtenu est un diamide : le N,N'-diméthyl-N,N'-adipamide de formule **(XIII).**

Une deuxième étape consiste en la réduction des fonctions amides du N,N'-diméthyl-N,N'-adipamide de formule **(XIII)** en fonctions amines tertiaires du pour conduire à la N,N'-diméthyl-1,6-hexanediamine (formule **(VI)**). Cette réduction peut s'effectuer par tous les moyens connus telle la réduction par hydrogénation catalytique ou l'action d'hydrures tels l'hydrure de lithium et d'aluminium.

Une troisième étape consiste à éthoxyler les fonctions amines secondaires de la N,N'-diméthyl-1,6-hexanediamine (formule **(VI)**) avec de l'oxyde d'éthylène (formule **(IV)**) pour conduire à la N,N'-diméthyl-N-N'-di(2-hydroxyéthyl)-1,6-hexanediamine (formule **(I₂)**).

Les méthodes 1 à 4 décrites ci-dessus permettent de synthétiser les composés selon la formule générale (I) à partir d'une famille de précurseurs ne contenant aucun atome d'halogène, qui ne produiront aucun acide hydrohalogéné. Les réactions mises en œuvre selon ces méthodes de synthèse génèrent aucun sel à séparer et à éliminer. Seuls des co-produits qui sont de l'eau et/ou des alcools légers sont générés. Contrairement aux méthodes impliquant des produits halogénés et générant des sels, ces méthodes de synthèse évitent la gestion et l'élimination d'une quantité importante de sels, et remplissent ainsi les conditions requises de nos jours en vue d'une chimie verte et durable, convenant à une production à une échelle industrielle.

### Méthode 5 : Synthèse à partir de 1,6-dihalohexane

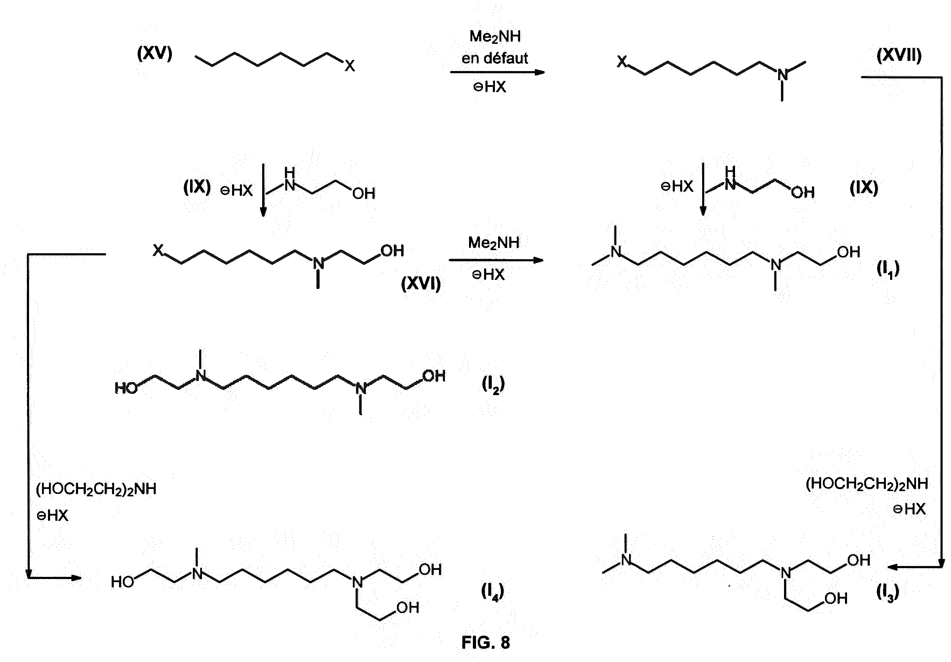

Un composé de formule générale **(I)** peut être synthétisé à partir d'un 1,6-dihalohexane (formule **(XV)**). La figure 8 illustre une telle méthode.

Dans la formule **(XV)**, X représente un atome de chlore, de brome ou d'iode. Le 1,6-dihalohexane peut ainsi être du 1,6-dichlorohexane, du 1,6-dibromohexane ou du 1,6-diiodohexane.

Tel que représenté à la figure 8, la N,N'-diméthyl-N,N'-di(2-hydroxyéthyl)-1,6-hexanediamine de formule **(I₂)** peut être obtenue par réaction de condensation de N-méthyl-2-aminoéthanol (formule **(IX)**) sur un 1,6-dihalohexane de formule **(XV).** Cette réaction peut aussi conduire au N-méthyl-N-(2-hydroxyéthyl)-1-halo-6-hexylamine (formule **(XVI)**).

Cette méthode peut être également appliquée à la synthèse de la N,N,N'-triméthyl-N'-(2-hydroxyéthyl)-1,6-hexanediamine (formule **(I₁)**) ou de la N,N-diméthyl-N'-N'-di(2-hydroxyéthyl)-1,6-hexanediamine(formule **(I₃)**) ou de la N-méthyl-N-N'-N'-tri(2-hydroxyéthyl)-1,6-hexanediamine (formule **(I₄)**) en effectuant la réaction en utilisant de manière adéquate, avec la N-méthyl-2-aminoéthanol (formule **(IX)**), la diméthylamine (Me₂NH) ou la diéthanolamine (HOCH₂CH₂)₂NH, successivement ou simultanément. Une autre voie consiste à effectuer la réaction de condensation en utilisant de manière adéquate, au lieu de la seule N-méthyl-2-aminoéthanol (formule **(IX)**), la diméthylamine (Me₂NH) et la diéthanolamine (HOCH₂CH₂)₂NH successivement ou simultanément. Le composé de formule (**(XVI)**) et la N,N-diméthyl-1-halo-6-hexylamine (formule (**XVII**)) peuvent être formés lors de ces réactions pour conduire à la N,N,N'-triméthyl-N'-(2-hydroxyéthyl)-1,6-hexanediamine (formule **(I₁)**), à la N,N-diméthyl-N'-N'-di(2-hydroxyéthyl)-1,6-hexanediamine(formule **(I₃)**) ou à la N-méthyl-N-N'-N'-tri(2-hydroxyéthyl)-1,6-hexanediamine (formule **(I₄)**).

### Nature des effluents gazeux

Selon l'invention, les solutions absorbantes peuvent être mises en œuvre pour désacidifier les effluents gazeux suivants : le gaz naturel, les gaz de synthèse, les fumées de combustion, les gaz de raffinerie, les gaz acides issus d'une unité aux amines, les gaz issus d'une unité de réduction en queue du procédé Claus, les gaz de fermentation de biomasse, les gaz de cimenterie, les fumées d'incinérateur. Ces effluents gazeux contiennent un ou plusieurs des composés acides suivants : le CO₂, l'H₂S, des mercaptans (par exemple le méthylmercaptan (CH₃SH), l'éthylmercaptan (CH₃CH₂SH), le propylmercaptan (CH₃CH₂CH₂SH)), du COS, du CS₂, le SO₂.

Les fumées de combustion sont produites notamment par la combustion d'hydrocarbures, de biogaz, de charbon dans une chaudière ou pour une turbine à gaz de combustion, par exemple dans le but de produire de l'électricité. A titre d'illustration, on peut mettre en œuvre un procédé de désacidification selon l'invention pour absorber au moins 70 %, de préférence au moins 80 % voire au moins 90 % du CO₂ contenu dans les fumées de combustion. Ces fumées ont généralement une température comprise entre 20°C et 60°C, une pression comprise entre 1 et 5 bar et peuvent comporter entre 50 % et 80 % d'azote, entre 5 % et 40 % de dioxyde de carbone, entre 1 % et 20 % d'oxygène, et quelques impuretés comme des SOx et des NOx, s'ils n'ont pas été éliminés en amont du procédé de désacidification. En particulier, le procédé de désacidification selon l'invention est particulièrement bien adapté pour absorber le CO₂ contenu dans des fumées de combustion comportant une faible pression partielle de CO₂, par exemple une pression partielle de CO₂ inférieure à 200 mbar.

Le procédé de désacidification selon l'invention peut être mis en œuvre pour désacidifier un gaz de synthèse. Le gaz de synthèse contient du monoxyde de carbone CO, de l'hydrogène H₂ (généralement dans un ratio H₂/CO égale à 2), de la vapeur d'eau (généralement à saturation à la température où le lavage est effectué) et du dioxyde de carbone CO₂ (de l'ordre de la dizaine de pourcents). La pression est généralement comprise entre 20 et 30 bar, mais peut atteindre jusqu'à 70 bar. Il peut contenir, en outre, des impuretés soufrées (H₂S, COS, etc.), azotées (NH₃, HCN) et halogénées.

Le procédé de désacidification selon l'invention peut être mis en œuvre pour désacidifier un gaz naturel. Le gaz naturel est constitué majoritairement d'hydrocarbures gazeux, mais peut contenir plusieurs des composés acides suivants : le CO₂, l'H₂S, des mercaptans, du COS, du CS₂. La teneur de ces composés acides est très variable et peut aller jusqu'à 70 % en volume pour le CO₂ et jusqu'à 40 % en volume pour l'H₂S. La température du gaz naturel peut être comprise entre 20°C et 100°C. La pression du gaz naturel à traiter peut être comprise entre 10 et 200 bar. L'invention peut être mise en œuvre pour atteindre des spécifications généralement imposées sur le gaz désacidifié, qui sont moins de 2 % de CO₂, voire moins de 50 ppm de CO₂ pour réaliser ensuite une liquéfaction du gaz naturel et moins de 4 ppm d'H₂S, et moins de 50 ppm, voire moins de 10 ppm, volume de soufre total.

### Procédé d'élimination des composés acides dans un effluent gazeux

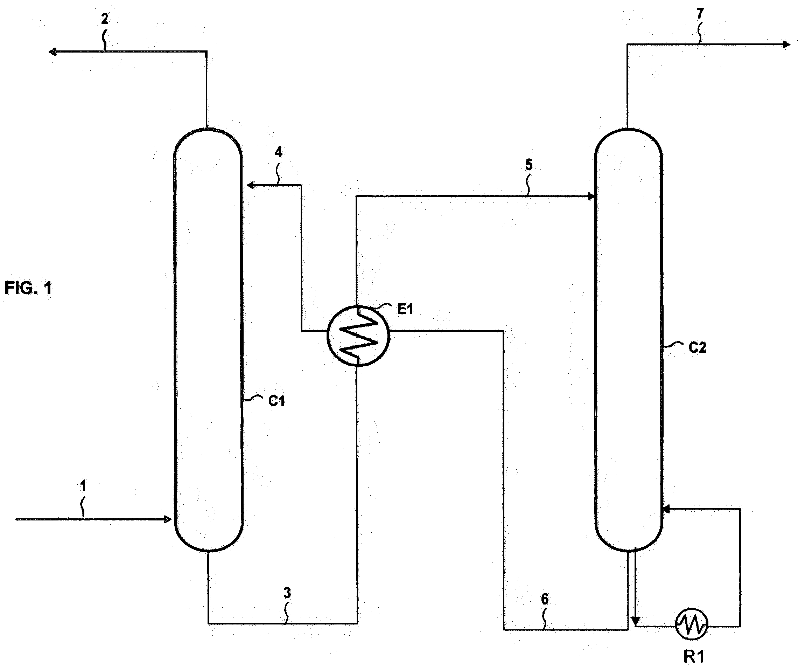

La mise en œuvre d'une solution aqueuse comportant au moins un composé azoté de formule générale (I), pour désacidifier un effluent gazeux est réalisée de façon schématique en effectuant une étape d'absorption suivie d'une étape de régénération, par exemple tel que représenté par la figure 1.

En référence à la figure 1, l'installation de désacidification d'un effluent gazeux selon l'invention comprend une colonne d'absorption C1 munie de moyens de mise en contact entre gaz et liquide, par exemple un garnissage vrac, un garnissage structuré ou des plateaux. L'effluent gazeux à traiter est acheminé par une conduite 1 débouchant au fond de la colonne C1. Une conduite 4 permet l'introduction de la solution absorbante en tête de la colonne C1. Une conduite 2 permet l'évacuation du gaz traité (désacidifié), et une conduite 3 permet d'acheminer la solution absorbante enrichie en composés acides suite à l'absorption vers une colonne de régénération C2. Cette colonne de régénération C2 est équipée d'internes de mise en contact entre gaz et liquide, par exemple des plateaux, des garnissages en vrac ou structurés. Le fond de la colonne C2 est équipé d'un rebouilleur R1 qui apporte la chaleur nécessaire à la régénération en vaporisant une fraction de la solution absorbante. La solution enrichie en composés acides est introduite en tête de la colonne de régénération C2 par une conduite 5. Une conduite 7 permet d'évacuer au sommet de la colonne C2 le gaz enrichi en composés acides libérés lors de la régénération, et une conduite 6 disposée au fond de la colonne C2 permet d'envoyer la solution absorbante régénérée vers la colonne d'absorption C1. Un échangeur de chaleur E1 permet de récupérer la chaleur de la solution absorbante régénérée issue de la colonne C2 pour chauffer la solution absorbante enrichie en composés acides sortant de la colonne d'absorption C1.

L'étape d'absorption consiste à mettre en contact l'effluent gazeux arrivant par la conduite 1 avec la solution absorbante arrivant par la conduite 4. Lors du contact, les fonctions amines des molécules de la solution absorbante réagissent avec les composés acides contenus dans l'effluent de manière à obtenir un effluent gazeux appauvri en composés acides qui est évacué par la conduite 2 en tête de la colonne C1 et une solution absorbante enrichie en composés acides évacuée par la conduite 3 en fond de la colonne C1 pour être régénérée.

L'étape d'absorption des composés acides peut être réalisée à une pression dans la colonne C1 comprise entre 1 bar et 200 bar, de préférence entre 20 bar et 100 bar pour le traitement d'un gaz naturel, de préférence entre 1 bar et 3 bar pour le traitement des fumées industrielles, et à une température dans la colonne C1 comprise entre 20°C et 100°C, préférentiellement comprise entre 30°C et 90°C, voire entre 30 et 60°C.

L'étape de régénération consiste notamment à chauffer et, éventuellement à détendre, la solution absorbante enrichie en composés acides afin de libérer les composés acides sous forme gazeuse. La solution absorbante enrichie en composés acides sortant de la colonne C1 est introduite dans l'échangeur de chaleur E1, où elle est réchauffée par le flux circulant dans la conduite 6 en provenance de la colonne de régénération C2. La solution réchauffée en sortie de E1 est introduite dans la colonne de régénération C2 par la conduite 5.

Dans la colonne de régénération C2, sous l'effet de la mise en contact de la solution absorbante arrivant par la conduite 5 avec la vapeur produite par le rebouilleur, les composés acides sont libérés sous forme gazeuse et évacués en tête de colonne C2 par la conduite 7. La solution absorbante régénérée, c'est-à-dire appauvrie en composés acides, est évacuée par la conduite 6 et est refroidie dans E1, puis recyclée dans la colonne d'absorption C1 par la conduite 4.

L'étape de régénération peut être réalisée par régénération thermique, éventuellement complétée par une ou plusieurs étapes de détente. Par exemple, la solution absorbante enrichie en composés acides évacuée par la conduite 3 peut être envoyée dans un premier ballon de détente (non représenté), avant son passage dans l'échangeur de chaleur E1. Dans le cas d'un gaz naturel, la détente permet d'obtenir un gaz évacué au sommet du ballon contenant la majeure partie des hydrocarbures aliphatiques co-absorbés par la solution absorbante. Ce gaz peut éventuellement être lavé par une fraction de la solution absorbante régénérée et le gaz ainsi obtenu peut être utilisé comme gaz combustible. Le ballon de détente opère de préférence à une pression inférieure à celle de la colonne d'absorption C1 et supérieure à celle de la colonne de régénération C2. Cette pression est généralement fixée par les conditions d'utilisation du gaz combustible, et est typiquement de l'ordre de 5 à 15 bar. Le ballon de détente opère à une température sensiblement identique à celle de la solution absorbante obtenue en fond de la colonne d'absorption C1.

La régénération peut être effectuée à une pression dans la colonne C2 comprise entre 1 bar et 5 bar, voire jusqu'à 10 bar et à une température dans la colonne C2 comprise entre 100°C et 180°C, de préférence comprise entre 110°C et 170°C, plus préférentiellement entre 120°C et 140°C. De manière préférée, la température de régénération dans la colonne C2 est comprise entre 155°C et 180°C dans le cas où l'on souhaite réinjecter les gaz acides. De manière préférée, la température de régénération dans la colonne C2 est comprise entre 115°C et 130°C dans les cas où le gaz acide est envoyé à l'atmosphère ou dans un procédé de traitement aval, comme un procédé Claus ou un procédé de traitement de gaz de queue.

### Exemples

Les exemples ci-dessous illustrent, de manière non limitative la synthèse de composés azotés de formule générale (I) ainsi que certaines des performances de ce composé lorsqu'il est utilisé en solution aqueuse pour éliminer des composés acides, comme le CO₂ ou l'H₂S, contenus dans un effluent gazeux par mise en contact de l'effluent gazeux avec la solution.

### Exemple 1 : synthèse de la N,N'-diméthyl-N-N'-di(2-hydroxyéthyl)-1,6-hexanediamine

A l'échelle du laboratoire, on réalise la synthèse de la N,N'-diméthyl-N-N'-di(2-hydroxyéthyl)-1,6-hexanediamine (formule **(I₂)**).

On fait réagir 160,5 g (1,035 mole) de 1,6-dichlorohexane avec 262,5 g (3,50 moles) de N-méthylaminoéthanol pendant 3 heures à 100°C. Après retour à la température ambiante, on neutralise le milieu avec une solution de 83,6 g de soude dans 240,0 g d'eau puis on procède à la filtration du sel formé. Après évaporation de l'eau et de l'excès de N-méthylaminoéthanol, on isole par distillation sous pression réduite 158,1 g d'un produit dont le spectre RMN-¹³C résumé ci-après est conforme à celui de la N,N'-diméthyl-N-N'-di(2-hydroxyéthyl)-1,6-hexanediamine.
26,2 ppm : HO-CH₂-CH₂-N(CH₃)-CH₂-**C**H₂-CH₂-CH₂-**C**H₂-CH₂-N(CH₃)-CH₂-CH₂-OH
26,5 ppm : HO-CH₂-CH₂-N(CH₃)-CH₂-CH₂-**C**H₂-**C**H₂-CH₂-CH₂-N(CH₃)-CH₂-CH₂-OH
41,3 ppm : HO-CH₂-CH₂-N(**C**H₃)-CH₂-CH₂-CH₂-CH₂-CH₂-CH₂-N(**C**H₃)-CH₂-CH₂-OH
57,2 ppm : HO-CH₂-CH₂-N(CH₃)-**C**H₂-CH₂-CH₂-CH₂-CH₂-**C**H₂-N(CH₃)-CH₂-CH₂-OH
58,0 ppm : HO-CH₂-**C**H₂-N(CH₃)-CH₂-CH₂-CH₂-CH₂-CH₂-CH₂-N(CH₃)-**C**H₂-CH₂-OH
58,5 ppm : HO-**C**H₂-CH₂-N(CH₃)-CH₂-CH₂-CH₂-CH₂-CH₂-CH₂-N(CH₃)-CH₂-**C**H₂-OH

### Exemple 2 : synthèse d'un mélange de N,N'-diméthyl-N-N'-di(2-hydroxyéthyl)-1,6-hexanediamine et de N,N,N'-triméthyl-N'-(2-hydroxyéthyl)-1,6-hexanediamine

On fait réagir dans un autoclave 155,0 g (1,0 mole) de 1,6-dichlorohexane avec 300,0 g (4,0 moles) de N-méthylaminoéthanol et 96,75 g d'une solution aqueuse de diméthylamine à 40% pendant 4 heures à 100°C. Après retour à la température ambiante, on neutralise le milieu avec une solution de 80,0 g de soude dans 80,0 g d'eau puis on procède à la filtration du sel formé. Après évaporation de l'eau et de l'excès de diméthylamine et de N-méthylaminoéthanol, on isole par distillation sous pression réduite 181,5 g d'un produit renfermant 73% de N,N'-diméthyl-N-N'-di(2-hydroxyéthyl)-1,6-hexanediamine (formule **(I₂)**) et 27% de N,N,N'-triméthyl-N'-(2-hydroxyéthyl)-1,6-hexanediamine (formule **(I₁)**).

### Exemple 3 : vitesse d'absorption du CO₂ d'une formulation d'amine pour un procédé d'absorption sélective.

On effectue des essais comparatifs d'absorption du CO₂ par différentes solutions absorbantes :
- une solution absorbante comprenant un mélange de N,N'-diméthyl-N-N'-di(2-hydroxyéthyl)-1,6-hexanediamine à 20% poids, de N-méthyldiéthanolamine (MDEA) à 30 % poids et d'eau à 50 % poids selon l'invention ;
- une solution aqueuse de MDEA à 47 % en poids de MDEA, qui constitue une solution absorbante de référence pour une élimination sélective en traitement de gaz ;

- une solution aqueuse de 1,2-bis-(pyrrolidinyléthoxy)-éthane à 50 % en poids de 1,2-bis-(pyrrolidinyléthoxy)-éthane, qui est un diaminoéther avec deux fonctions amines tertiaires selon la formule générale du brevet US 4,405,582, mais qui ne possède pas de fonction alcool et qui n'entre pas dans la formule générale (I) ;
- une solution aqueuse de 1,2-bis-(tertiobutylaminoéthoxy)-éthane à 40 % en poids de 1,2-bis-(tertiobutylaminoéthoxy)-éthane, qui est un diaminoéther avec deux fonctions secondaires ayant un encombrement stérique sévère des atomes d'azote selon la formule générale du brevet US 4,405,583, sans fonction alcool et qui n'entre pas dans la formule générale (I);
- une solution aqueuse de N,N,N',N'-tétraméthyl-1,6-hexanediamine (TMHDA) à 50 % en poids de TMHDA, qui est une diamine tertiaire divulguée dans le brevet FR2934172, mais qui ne possède pas de fonction alcool et qui n'entre pas dans la formule générale (I).

Pour chaque essai, on mesure le flux d'absorption du CO₂ par la solution absorbante aqueuse dans un réacteur fermé, du type cellule de Lewis. 200 g de solution est introduite dans le réacteur fermé, régulé à une température de 50°C. On réalise quatre injections successives de CO₂ de 100 à 200 mbar dans la phase vapeur du réacteur ayant un volume de 200 cm³. La phase gaz et la phase liquide sont agitées à 100 tours/minutes et entièrement caractérisées du point de vue hydrodynamique. Pour chaque injection, on mesure la vitesse d'absorption du CO₂ par variation de pression dans la phase gaz. On détermine ainsi un coefficient de transfert global Kg par une moyenne des résultats obtenus sur les quatre injections.

Les résultats obtenus sont présentés dans le tableau 1 ci-dessous en vitesse d'absorption relative par rapport à la solution absorbante aqueuse de référence comprenant 47 % poids de MDEA, cette vitesse d'absorption relative étant définie par le rapport du coefficient de transfert global de la solution absorbante testée sur le coefficient de transfert global de la solution absorbante de référence (avec MDEA).

**Tableau 1**

| Composés | Concentration totale en amine (% en poids) | Vitesse d'absorption relative du CO₂ à 50°C |
|---|---|---|
| MDEA | 47 | 1,00 |
| 1,2-bis-(pyrrolidinyléthoxy)-éthane (selon le brevet US 4,405,582) | 50 | 1,43 |
| 1,2-bis-(tertiobutylaminoéthoxy)-éthane (selon le brevet US 4,405,583) | 40 | 1,74 |
| TMHDA (selon FR2934172) | 50 | 2,72 |
| N,N'-diméthyl-N-N'-di(2-hydroxyéthyl)-1,6-hexanediamine 20 % poids + MDEA 30% poids selon l'invention | 50 | 0,94 |

L'examen des résultats fait ressortir, dans ces conditions de test, une vitesse d'absorption du CO₂ par la solution absorbante selon l'invention plus lente par rapport à la formulation de référence avec la MDEA, et par rapport aux solutions absorbantes avec certaines molécules de l'art antérieur. Il apparaît donc que le composé exemplifié selon l'invention présente étonnamment un intérêt particulier et amélioré dans le cas d'une désacidification sélective d'un effluent gazeux dans laquelle on cherche à limiter la cinétique d'absorption du CO₂.

### Exemple 4 : capacité d'absorption de l'H₂S d'une formulation de N,N'-diméthyl-N-N'-di(2-hydroxyéthyl)-1,6-hexanediamine pour un procédé de traitement de gaz acide.

Les performances de capacité d'absorption de l'H₂S à 40°C d'une solution aqueuse de N,N'-diméthyl-N-N'-di(2-hydroxyéthyl)-1,6-hexanediamine selon l'invention, contenant 20 % en poids de N,N'-diméthyl-N-N'-di(2-hydroxyéthyl)-1,6-hexanediamine et 30% en poids de MDEA, est comparée à celles d'une solution aqueuse de MDEA contenant 50 % en poids de MDEA, qui constitue une solution absorbante de référence pour la désacidification de gaz contenant de l'H₂S.

On réalise un test d'absorption à 40 °C sur des solutions aqueuses d'amine au sein d'une cellule d'équilibre thermostatée. Ce test consiste à injecter dans la cellule d'équilibre, préalablement remplie de solution aqueuse d'amine dégazée, une quantité connue de gaz acide, de l'H₂S dans cet exemple, puis à attendre l'établissement de l'état d'équilibre. Les quantités de gaz acide absorbées dans la solution aqueuse d'amine sont alors déduites des mesures de température et de pression grâce à des bilans de matière et de volume. Les solubilités sont représentées de manière classique sous la forme des pressions partielles en H₂S (en bar) en fonction du taux de charge en H₂S (en mol d' H₂S/kg de solution absorbante et en mol d' H₂S/mol d'amine).

Dans le cas d'une désacidification en traitement de gaz naturel, les pressions partielles en H₂S rencontrées dans les gaz acides sont typiquement comprises entre 0,1 et 1 bar, à une température de 40°C. A titre d'exemple, dans cette gamme industrielle, on compare dans le tableau 2 ci-dessous les taux de charge d'H₂S obtenus à 40°C pour différentes pressions partielles en H₂S entre la solution absorbante de MDEA à 50 % poids et la solution absorbante de N,N'-diméthyl-N-N'-di(2-hydroxyéthyl)-1,6-hexanediamine selon l'invention, contenant 20 % en poids de N,N'-diméthyl-N-N'-di(2-hydroxyéthyl)-1,6-hexanediamine et 30% en poids de MDEA.

**Tableau 2**

| | N,N'-diméthyl-N-N'-di(2-hydroxyéthyl)-1,6-hexanediamine 20% poids + MDEA 30% poids selon l'invention à 40 °C | | Solution aqueuse de MDEA à 50% poids à 40 °C | |
|---|---|---|---|---|
| Pression partielle en H₂S (bar) | Taux de charge en H₂S (mol/mol d'amine) | Taux de charge en H₂S (mol/kg) | Taux de charge en H₂S (mol/mol d'amine) | Taux de charge en H₂S (mol/kg) |
| 0,10 | 0,41 | 1,39 | 0,21 | 0,88 |
| 1 | 0,99 | 3,34 | 0,69 | 2,95 |

A 40 °C, quelle que soit la pression partielle en H₂S, la capacité d'absorption de la solution aqueuse de N,N'-diméthyl-N-N'-di(2-hydroxyéthyl)-1,6-hexanediamine selon l'invention est supérieure à celle de la solution de MDEA.

A une pression partielle en H₂S de 0,10 bar, l'écart entre les taux de charge en H₂S des deux solutions absorbantes s'élève à 0,51 mol/kg avec une capacité d'absorption pour la solution absorbante de N,N'-diméthyl-N-N'-di(2-hydroxyéthyl)-1,6-hexanediamine augmentée de 58% par rapport à la solution absorbante de MDEA de référence.

A une pression partielle en H₂S de 1 bar, l'augmentation de taux de charge en H₂S pour la solution absorbante de N,N'-diméthyl-N-N'-di(2-hydroxyéthyl)-1,6-hexanediamine est encore de 13% par rapport à la solution absorbante de MDEA de référence.

On constate donc que la solution aqueuse de N,N'-diméthyl-N-N'-di(2-hydroxyéthyl)-1,6-hexanediamine selon l'invention a une capacité d'absorption de l'H₂S plus élevée que la solution aqueuse de MDEA de référence à 50 % poids de MDEA à 40°C, dans la gamme de pressions partielles en H₂S comprise entre 0,1 et 1 bar correspondant à une gamme de pression partielle représentative des conditions industrielles usuelles.

Il apparaît donc que cette molécule exemplifiée selon l'invention permet de réduire les débits de solution absorbante à mettre en œuvre sur des applications de désacidification de gaz contenant H₂S par rapport à la solution absorbante de MDEA de référence.

L'absorption du CO₂ étant plus lente dans une solution aqueuse de N,N'-diméthyl-N-N'-di(2-hydroxyéthyl)-1,6-hexanediamine selon l'invention que dans une solution aqueuse de MDEA (voir exemple 3 ci-dessus) et la capacité d'absorption équivalente ou supérieure en gaz acides, notamment en H₂S, de la solution absorbante de N,N'-diméthyl-N-N'-di(2-hydroxyéthyl)-1,6-hexanediamine par rapport à une solution aqueuse de MDEA comme illustré dans le présent exemple, il apparaît que cette molécule exemplifiée selon l'invention permet de réduire les débits de solution absorbante à mettre en œuvre sur des applications de désacidification sélective (H₂S par rapport au CO₂) pour absorber un débit donné de H₂S tout en réduisant le débit de CO₂ co-absorbé par rapport à la solution absorbante de MDEA de référence.

### Exemple 5 : capacité d'absorption du CO₂ d'une formulation de N,N'-diméthyl-N-N'-di(2-hvdroxvéthvl)-1,6-hexanediamine pour une application de désacidification totale en traitement de gaz acide.

Les performances de capacité d'absorption du CO₂ à 40 °C d'une solution aqueuse de N,N'-diméthyl-N-N'-di(2-hydroxyéthyl)-1,6-hexanediamine selon l'invention, contenant 42 % en poids de N,N'-diméthyl-N-N'-di(2-hydroxyéthyl)-1,6-hexanediamine et 5,3% de pipérazine est comparée à celles d'une solution aqueuse de MDEA contenant 39 % en poids de MDEA et 6,7% de pipérazine, qui constitue une solution absorbante de référence pour une application de désacidification totale.

On réalise un test d'absorption à 40 °C selon le mode opératoire décrit dans les exemples précédents, le gaz acide étant du CO₂ au lieu de l'H₂S.

Dans le cas d'une désacidification en traitement de gaz naturel, les pressions partielles en CO₂ rencontrées dans les gaz acides sont typiquement comprises entre 0,1 et 1 bar, à une température de 40°C. A titre d'exemple, dans cette gamme industrielle, on compare dans le tableau 3 ci-dessous les taux de charge en CO₂ obtenus à 40°C pour différentes pressions partielles en CO₂ entre la solution absorbante de MDEA à 39 % poids et 6,7% poids de pipérazine et la solution absorbante de N,N'-diméthyl-N-N'-di(2-hydroxyéthyl)-1,6-hexanediamine à 42 % poids et 5,3% poids de pipérazine.

**Tableau 3**

| | Solution aqueuse de N,N'-diméthyl-N-N'-di(2-hydroxyéthyl)-1,6-hexanediamine à 42 % poids et 5,3% poids de pipérazine à 40 °C | | Solution aqueuse de MDEA à 39 % poids et 6,7%poids de pipérazine à 40 °C | |
|---|---|---|---|---|
| Pression partielle en CO₂ (bar) | Taux de charge en CO₂ (mol/mol d'amine) | Taux de charge en CO₂ (mol/kg) | Taux de charge en CO₂ (mol/mol d'amine) | Taux de charge en CO₂ (mol/kg) |
| 0,10 | 0,88 | 2,14 | 0,39 | 1,60 |
| 1 | 1,48 | 3,58 | 0,73 | 2,96 |

A 40 °C, quelle que soit la pression partielle en CO₂, la capacité d'absorption de la solution aqueuse de N,N'-diméthyl-N-N'-di(2-hydroxyéthyl)-1,6-hexanediamine activée avec 5,3% de pipérazine selon l'invention est supérieure à celle de la solution de MDEA activée avec 6,7% poids de pipérazine.

A une pression partielle en CO₂ de 0,10 bar, l'écart entre les taux de charge en CO₂ des deux solutions absorbantes s'élève à 0,54 mol/kg avec une capacité d'absorption pour la solution absorbante de N,N'-diméthyl-N-N'-di(2-hydroxyéthyl)-1,6-hexanediamine augmentée de 34% par rapport à la solution absorbante de MDEA activée de référence.

A une pression partielle en CO₂ de 1 bar, l'augmentation de taux de charge en CO₂ pour la solution absorbante de N,N'-diméthyl-N-N'-di(2-hydroxyéthyl)-1,6-hexanediamine activée est encore de 21% par rapport à la solution absorbante de MDEA activée de référence.

On constate donc que la solution aqueuse de N,N'-diméthyl-N-N'-di(2-hydroxyéthyl)-1,6-hexanediamine à 42 % poids activée avec 5,3% de pipérazine a une capacité d'absorption du CO₂ à 40°C plus élevée que la solution aqueuse de MDEA de référence à 39 % poids de MDEA activée avec 6,7% poids de pipérazine, dans la gamme de pressions partielles en CO₂ comprise entre 0,1 et 1 bar correspondant à une gamme de pression partielle représentative des conditions industrielles usuelles.

Il apparaît donc que cette molécule exemplifiée selon l'invention permet de réduire les débits de solution absorbante à mettre en œuvre sur des applications de désacidification de gaz contenant du CO₂ par rapport à la solution absorbante de MDEA activée de référence.

## Revendications

1. Procédé d'élimination des composés acides contenus dans un effluent gazeux dans lequel on effectue une étape d'absorption des composés acides par mise en contact de l'effluent gazeux avec une solution absorbante comportant :
- de l'eau ;
- au moins un composé azoté répondant à la formule générale (I) suivante : 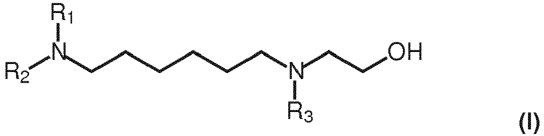 dans laquelle :
les radicaux R₁, R₂, R₃ sont chacun choisi indifféremment parmi un radical méthyle et un radical hydroxyéthyle, et
au moins un radical parmi R₁, R₂, et R₃ est un radical méthyle.

2. Procédé selon la revendication 1, dans lequel ledit au moins composé est choisi parmi les composés suivants :
- la N,N,N'-triméthyl-N'-(2-hydroxyéthyl)-1,6-hexanediamine de formule (I₁) suivante : 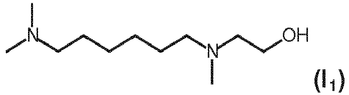
- la N,N'-diméthyl-N,N'-di(2-hydroxyéthyl)-1,6-hexanediamine de formule (I₂) : 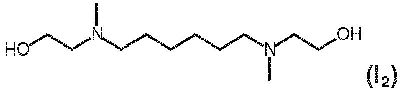
- la N,N-diméthyl-N',N'-di(2-hydroxyéthyl)-1,6-hexanediamine de formule (I₃) : 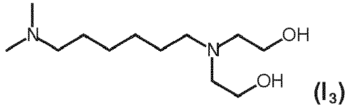
- la N-méthyl-N,N',N'-tri(2-hydroxyéthyl)-1,6-hexanediamine de formule (I₄) :

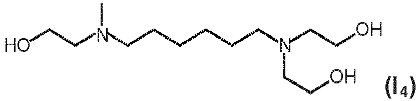

3. Procédé selon la revendication 2, dans lequel ledit composé est la N,N,N'-triméthyl-N'-(2-hydroxyéthyl)-1,6-hexanediamine de formule **(I₁)** ou la N,N'-diméthyl-N,N'-di(2-hydroxyéthyl)-1,6-hexanediamine de formule **(I₂).**

4. Procédé selon l'une des revendications précédentes, dans lequel la solution absorbante comporte entre 5 % et 95 % poids dudit composé azoté, de préférence entre 10 % et 90 % poids dudit composé azoté, et entre 5 % et 95 % poids d'eau, et de préférence entre 10 % et 90% poids d'eau.

5. Procédé selon la revendication 4, dans lequel la solution absorbante comporte entre 20 % et 50 % poids dudit composé azoté, et entre 50 % et 80 % poids d'eau.

6. Procédé selon l'une des revendications précédentes, dans lequel la solution absorbante comporte en outre entre 5 % et 95 % poids d'au moins une amine supplémentaire, ladite amine supplémentaire étant soit une amine tertiaire, soit une amine secondaire comprenant deux carbones secondaires en alpha de l'atome d'azote ou au moins un carbone tertiaire en alpha de l'atome d'azote.

7. Procédé selon la revendication 6, dans lequel ladite amine supplémentaire est une amine tertiaire choisie dans le groupe constitué par :
- la N-méthyldiéthanolamine ;
- la triéthanolamine ;
- la diéthylmonoéthanolamine ;
- la diméthylmonoéthanolamine ; et
- l'éthyldiéthanolamine.

8. Procédé selon l'une des revendications précédentes, dans lequel la solution absorbante comporte en outre une quantité non nulle et inférieure à 30 % poids d'au moins une amine primaire ou une amine secondaire.

9. Procédé selon la revendication 8, dans lequel ladite amine primaire ou secondaire est choisie dans le groupe constitué par :
- la monoéthanolamine ;
- la diéthanolamine ;
- la N-butyléthanolamine ;
- l'aminoéthyléthanolamine ;
- le diglycolamine ;
- la pipérazine ;
- la 1-méthyl-pipérazine ;
- la 2-méthyl-pipérazine ;
- l'homopipérazine ;
- la N-(2-hydroxyéthyl)pipérazine ;
- la N-(2-aminoéthyl)pipérazine ;
- la morpholine ;
- la 3-(méthylamino)propylamine ;
- la 1,6-hexanediamine ;
- la N,N-diméthyl-1,6-hexanediamine ;
- la N,N'-diméthyl-1,6-hexanediamine
- la N-méthyl-1,6-hexanediamine ; et
- la N,N',N'-triméthyl-1,6-hexanediamine.

10. Procédé selon l'une des revendications précédentes, dans lequel la solution absorbante comporte en outre au moins un solvant physique choisi dans le groupe constitué par le méthanol, l'éthanol, le 2-éthoxyéthanol, le triéthylèneglycoldiméthyléther, le tétraéthylèneglycoldiméthyléther, le pentaéthylèneglycoldiméthyléther, l'hexaéthylèneglycoldiméthyléther, l'heptaéthylèneglycoldiméthyléther, l'octaéthylèneglycoldiméthyléther, le butoxyacétate de diéthylèneglycol, le triacétate de glycérol, le sulfolane, la N-méthylpyrrolidone, la N-méthylmorpholin-3-one, le N,N-diméthylformamide, la N-formyl-morpholine, la N,N-diméthyl-imidazolidin-2-one, le N-méthylimidazole, l'éthylèneglycol, le diéthylèneglycol, le triéthylèneglycol, le thiodiglycol, le carbonate de propylène, le tributylphosphate.

11. Procédé selon l'une des revendications précédentes, dans lequel l'étape d'absorption des composés acides est réalisée à une pression comprise entre 1 bar et 200 bar, et à une température comprise entre 20°C et 100°C.

12. Procédé selon l'une des revendications précédentes, dans lequel on obtient une solution absorbante chargée en composés acides après l'étape d'absorption, et on effectue au moins une étape de régénération de ladite solution absorbante chargée en composés acides à une pression comprise entre 1 bar et 10 bar et à une température comprise entre 100 °C et 180 °C.

13. Procédé selon l'une des revendications précédentes, dans lequel l'effluent gazeux est choisi parmi le gaz naturel, les gaz de synthèse, les fumées de combustion, les gaz de raffinerie, les gaz acides issus d'une unité aux amines, les gaz issus d'une unité de réduction en queue du procédé Claus, les gaz de fermentation de biomasse, les gaz de cimenterie, les fumées d'incinérateur.

14. Procédé selon l'une des revendications précédentes, mis en œuvre pour l'élimination sélective de l'H₂S par rapport au CO₂ d'un effluent gazeux comportant de l'H₂S et du CO₂, de préférence du gaz naturel.

## Patentansprüche

1. Verfahren zur Eliminierung von sauren Verbindungen in einem Gasabstrom, wobei ein Schritt der Absorption der sauren Verbindungen durch Inberührungbringen des Gasabstroms mit einer absorbierenden Lösung durchgeführt wird, umfassend:
- Wasser;
- mindestens eine Stickstoffverbindung mit der folgenden allgemeinen Formel (I): 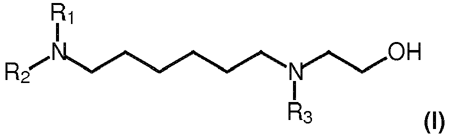 wobei:
die Reste R₁, R₂, R₃ jeweils unabhängig ausgewählt werden aus einem Methylrest und einem Hydroxyethylrest, und
mindestens ein Rest von R₁, R₂, R₃ ein Methylrest ist.

2. Verfahren nach Anspruch 1, wobei mindestens eine Verbindung ausgewählt wird aus den folgenden Verbindungen:
- N,N,N'-Trimethyl-N'-(2-hydroxyethyl)-1,6-hexandiamin mit der folgenden Formel (I₁): 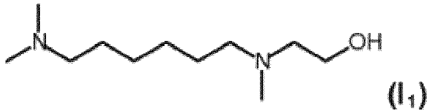
- N,N'-Dimethyl-N,N'-di-(2-hydroxyethyl)-1,6-hexandiamin mit der Formel (I₂): 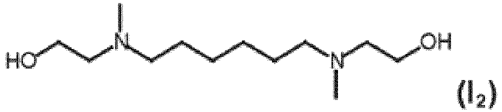
- N,N-Dimethyl-N',N'-di-(2-hydroxyethyl)-1,6-hexandiamin mit der Formel (I₃): 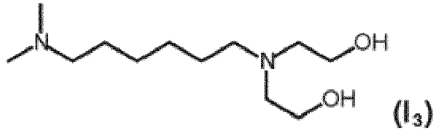
- N-Methyl-N,N',N'-tri-(2-hydroxyethyl)-1,6-hexandiamin mit der Formel (I₄):

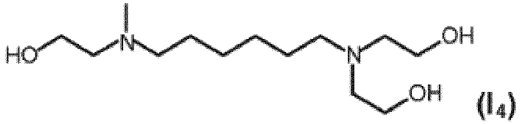

3. Verfahren nach Anspruch 2, wobei die Verbindung N,N,N'-Trimethyl-N'-(2-hydroxyethyl)-1,6-hexandiamin mit der Formel (I₁) oder N,N'-Dimethyl-N,N'-di-(2-hydroxyethyl)-1,6-hexandiamin mit der Formel (I₂) ist.

4. Verfahren nach einem der vorhergehenden Ansprüche, wobei die absorbierende Lösung zwischen 5 Gew.-% und 95 Gew.-% der Stickstoffverbindung, vorzugsweise zwischen 10 Gew.-% und 90 Gew.-% der Stickstoffverbindung, und zwischen 5 Gew.-% und 95 Gew.-% Wasser, und vorzugsweise zwischen 10 Gew.-% und 90 Gew.-% Wasser, umfasst.

5. Verfahren nach Anspruch 4, wobei die absorbierende Lösung zwischen 20 Gew.-% und 50 Gew.-% der Stickstoffverbindung, und zwischen 50 Gew.-% und 80 Gew.-% Wasser umfasst.

6. Verfahren nach einem der vorhergehenden Ansprüche, wobei die absorbierende Lösung außerdem zwischen 5 Gew.-% und 95 Gew.-% mindestens eines ergänzenden Amins umfasst, wobei das ergänzende Amin entweder ein tertiäres Amin oder ein sekundäres Amin ist, umfassend zwei sekundäre Kohlenstoffe in alpha des Stickstoffatoms oder mindestens einen tertiären Kohlenstoff in alpha des Stickstoffatoms.

7. Verfahren nach Anspruch 6, wobei das ergänzende Amin ein tertiäres Amin ist, das ausgewählt wird aus der Gruppe bestehend aus:
- N-Methyldiethanolamin;
- Triethanolamin;
- Diethylmonoethanolamin;
- Dimethylmonoethanolamin; und
- Ethyldiethanolamin.

8. Verfahren nach einem der vorhergehenden Ansprüche, wobei die absorbierende Lösung außerdem eine Menge von nicht Null und kleiner als 30 Gew.-% mindestens eines primären Amins oder eines sekundären Amins umfasst.

9. Verfahren nach Anspruch 8, wobei das primäre oder sekundäre Amin ausgewählt wird aus der Gruppe bestehend aus:
- Monoethanolamin;
- Diethanolamin;
- N-Butylethanolamin;
- Aminoethylethanolamin;
- Diglykolamin;
- Piperazin;
- 1-Methylpiperazin;
- 2-Methylpiperazin;
- Homopiperazin;
- N-(2-hydroxyethyl)-piperazin;
- N-(2-aminoethyl)-piperazin;
- Morpholin;
- 3-(Methylamino)-propylamin;
- 1,6-Hexandiamin;
- N,N-Dimethyl-1,6-hexandiamin;
- N,N'-Dimethyl-1,6-hexandiamin;
- N-Methyl-1,6-hexandiamin; und
- N,N',N'-Trimethyl-1,6-hexandiamin.

10. Verfahren nach einem der vorhergehenden Ansprüche, wobei die adsorbierende Lösung außerdem mindestens ein physikalisches Lösungsmittel umfasst, das ausgewählt wird aus der Gruppe bestehend aus Methanol, Ethanol, 2-Ethoxyethanol, Triethylenglykoldimethylether, Tetraethylenglykoldimethylether, Pentaethylenglykoldimethylether, Hexaethylenglykoldimethylether, Heptaethylenglykoldimethylether, Octaethylenglykoldimethylether, Diethylenglykolbutoxyacetat, Glycerintriacetat, Sulfolan, N-Methylpyrrolidon, N-Methylmorpholin-3-on, N,N-Dimethylformamid, N-Formylmorpholin, N,N-Dimethylimidazolidin-2-on, N-Methylimidazol, Ethylenglykol, Diethylenglykol, Triethylenglykol, Thiodiglykol, Propylencarbonat, Tributylphosphat.

11. Verfahren nach einem der vorhergehenden Ansprüche, wobei der Schritt der Absorption der sauren Verbindungen bei einem Druck zwischen 1 bar und 200 bar und bei einer Temperatur zwischen 20 °C und 100 °C durchgeführt wird.

12. Verfahren nach einem der vorhergehenden Ansprüche, wobei eine absorbierende Lösung, die mit sauren Verbindungen beladen ist, nach dem Absorptionsschritt erhalten wird, und mindestens ein Regenerierungsschritt der absorbierenden Lösung, die mit sauren Verbindungen beladen ist, bei einem Druck zwischen 1 bar und 10 bar und bei einer Temperatur zwischen 100 °C und 180 °C durchgeführt wird.

13. Verfahren nach einem der vorhergehenden Ansprüche, wobei der Gasabstrom ausgewählt wird aus Erdgas, Synthesegasen, Verbrennungsgasen, Raffineriegasen, sauren Gasen aus einer Amin-Einheit, Gasen aus einer Stufenreduktionseinheit des Claus-Prozesses, Biomassefermentationsgasen, Zementfabriksgasen, Verbrennungsanlagengasen.

14. Verfahren nach einem der vorhergehenden Ansprüche, welches zur selektiven Eliminierung von H₂S im Verhältnis zu CO₂ aus einem Gasabstrom verwendet wird, umfassend H₂S und CO₂, vorzugsweise Erdgas.

## Claims

1. A method of removing acid compounds contained in a gaseous effluent, wherein an acid compound absorption step is carried out by contacting the gaseous effluent with an absorbent solution comprising:
- water,
- at least one nitrogen compound with general formula (I) as follows: 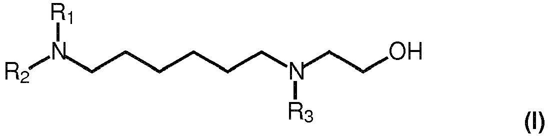 wherein:
radicals R1, R2, R3 are each selected indiscriminately among a methyl radical and a hydroxyethyl radical, and
at least one radical among R₁, R₂, R₃ is a methyl radical.

2. A method as claimed in claim 1, wherein said at least one compound is selected from the following compounds:
- N,N,N'-trimethyl-N'-(2-hydroxyethyl)-1,6-hexanediamine with formula (I₁) as follows: 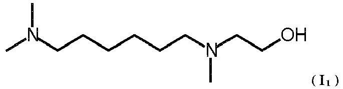
- N,N'-dimethyl-N,N'-di(2-hydroxyethyl)-1,6-hexanediamine with formula (I2): 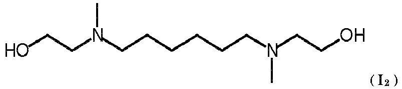
- N,N-dimethyl-N',N'-di(2-hydroxyethyl)-1,6-hexanediamine with formula (I3) : 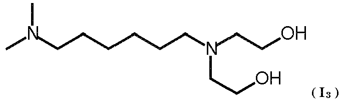
- N-methyl-N,N',N'-tri(2-hydroxyethyl)-1,6-hexanediamine with formula (I4):

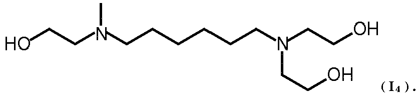

3. A method as claimed in claim 2, wherein said compound is N,N,N'-trimethyl-N'-(2-hydroxyethyl)-1,6-hexanediamine with formula (I₁) or N,N'-dimethyl-N,N'-di(2-hydroxyethyl)-1,6-hexanediamine with formula (I2).

4. A method as claimed in any one of the previous claims, wherein the absorbent solution comprises between 5 wt.% and 95 wt.% of said nitrogen compound, preferably between 10 wt.% and 90 wt.% of said nitrogen compound, and between 5 wt.% and 95 wt.% of water, preferably between 10 wt.% and 90 wt.% of water.

5. A method as claimed in claim 4, wherein the absorbent solution comprises between 20 wt.% and 50 wt.% of said nitrogen compound, and between 50 wt.% and 80 wt.% of water.

6. A method as claimed in any one of the previous claims, wherein the absorbent solution further comprises between 5 wt.% and 95 wt.% of at least one additional amine, said additional amine being either a tertiary amine or a secondary amine having two secondary carbons at nitrogen alpha position or at least one tertiary carbon at nitrogen alpha position.

7. A method as claimed in claim 6, wherein said additional amine is a tertiary amine selected from the group made up of:
- N-methyldiethanolamine,
- triethanolamine,
- diethylmonoethanolamine,
- dimethylmonoethanolamine, and
- ethyldiethanolamine.

8. A method as claimed in any one of the previous claims, wherein the absorbent solution further comprises a non-zero amount, less than 30 wt.%, of at least one primary or secondary amine.

9. A method as claimed in claim 8, wherein said primary or secondary amine is selected from the group made up of:
- monoethanolamine,
- diethanolamine,
- N-butylethanolamine,
- aminoethylethanolamine,
- diglycolamine,
- piperazine,
- 1-methylpiperazine,
- 2-methylpiperazine,
- homopiperazine,
- N-(2-hydroxyethyl)piperazine,
- N-(2-aminoethyl)piperazine,
- morpholine,
- 3-(methylamino)propylamine,
- 1,6-hexanediamine,
- N,N-dimethyl-1,6-hexanediamine,
- N,N'-dimethyl-1,6-hexanediamine,
- N-methyl-1,6-hexanediamine, and
- N,N',N'-trimethyl-1,6-hexanediamine.

10. A method as claimed in any one of the previous claims, wherein the absorbent solution further comprises at least one physical solvent selected from the group made up of methanol, ethanol, 2-ethoxyethanol, triethylene glycol dimethyl ether, tetraethylene glycol dimethyl ether, pentaethylene glycol dimethyl ether, hexaethylene glycol dimethyl ether, heptaethylene glycol dimethyl ether, octaethylene glycol dimethyl ether, diethylene glycol butoxyacetate, glycerol triacetate, sulfolane, N-methyl-pyrrolidone, N-methylmorpholin-3-one, N,N-dimethylformamide, N-formylmorpholine, N,N-dimethyl-imidazolidin-2-one, N-methylimidazole, ethylene glycol, diethylene glycol, triethylene glycol, thiodiglycol, propylene carbonate and tributyl phosphate.

11. A method as claimed in any one of the previous claims, wherein the acid compound absorption step is carried out at a pressure ranging between 1 bar and 200 bar, and at a temperature ranging between 20°C and 100°C.

12. A method as claimed in any one of the previous claims, wherein an absorbent solution laden with acid compounds is obtained after the absorption step and at least one step of regenerating said acid compound-laden absorbent solution is performed at a pressure ranging between 1 bar and 10 bar, and at a temperature ranging between 100°C and 180°C.

13. A method as claimed in any one of the previous claims, wherein the gaseous effluent is selected among natural gas, syngases, combustion fumes, refinery gas, acid gas from an amine plant, Claus tail gas, biomass fermentation gas, cement plant gas and incinerator fumes.

14. A method as claimed any one of the previous claims, implemented for selectively removing the H₂S over the CO₂ from a gaseous effluent comprising H₂S and CO₂, preferably natural gas.
